# EUROPEAN PATENT SPECIFICATION

(11) **EP 1 562 494 B1**
(45) Date of publication and mention of the grant of the patent: **20.04.2011**
(21) Application number: 03749799.7
(22) Date of filing: 01.09.2003
(51) Int. Cl.: A61B 17/32

(54) **DISPOSABLE SCALPEL WITH RETRACTABLE BLADE**
EINWEGSKALPELL MIT EINZIEHBARER KLINGE
SCALPEL JETABLE A LAME RETRACTABLE

(30) Priority: 18.11.2002 ZA 200209334; 11.07.2003 ZA 200305355
(43) Date of publication of application: 17.08.2005
(73) Proprietor: Milton, Trevor John, 2196 Johannesburg (Gauteng Province) (ZA)
(72) Inventor: Milton, Trevor John, 2196 Johannesburg (Gauteng Province) (ZA); Holman, Robert Gerard, Randburg 2125 (ZA)
(74) Representative: Jones, Graham Henry
(86) International application number: PCT/ZA2003/000126
(87) International publication number: WO 2004/045428

(56) References cited:
- US-A- 5 431 672
- US-A- 5 599 351
- US-B1- 6 254 621
- US-B1- 6 314 646
- US-B1- 6 391 041

## Description

### FIELD OF THE INVENTION

This invention relates to a disposable scalpel having a retractable blade and, more particularly, to a disposable scalpel in which movement of the blade relative to a supporting scalpel handle between an extended operative position and a retracted inoperative position is achieved by moving a blade carrier by way of a thumb operable slider attached, generally by way of a slot through the wall of the handle, to the blade carrier.

### BACKGROUND TO THE INVENTION

Disposable scalpels having retractable blades have been proposed and produced in many different forms. The various different forms can, for present purposes, be considered to fall into two different categories; a first being scalpels in which the thumb operable slider projects through a side wall of the scalpel handle, and a second in which the slider projects through a slot in one edge that can be considered to be the top edge of the scalpel handle.

The first type of scalpel that has the slider projecting through a side wall of the handle is considered to suffer from a number of disadvantages, not least of which is that one designed for use by a right-handed person cannot easily be used by a left-handed person and vice versa. United States patent No 6,254,621 describes a scalpel that is typical of this type.

The second type of scalpel that has the slider projecting through the top edge of the scalpel handle generally has the disadvantage that the scalpel handle is made in two Darts that are subsequently secured together with the blade carrier inside the handle and the associated slider projecting through a slot in the top edge of the composite handle. Typical of this type of construction are the scalpels described in United States patents 5,330,493; 5,556,409; and 5,571,127. The two-part construction of the handle is considered by applicant to be undesirable for a variety of reasons not least of which is the fact that the handle could possibly come apart.

A one-part construction of a utility knife is shown in US-B-6314646.

There are a number of factors that are independent of the type of construction that are considered to be desirable and that are present to greater or lesser extents in existing scalpels, these being factors that contribute to the scalpel blade being held firmly in its operative position: being held positively in Its retracted inoperative position; and also a facility aimed at preventing reuse of a scalpel in an effort to avoid so-called sharps injuries to personnel that may come into contact with used medical equipment.

### OBJECT OF THE INVENTION

It is, accordingly, an object of this invention to provide a scalpel with a retractable blade that has one or more improved features over the prior art scalpels of which applicant is aware.

### SUMMARY OF THE INVENTION

In accordance with this invention there is provided a scalpel having a handle with a longitudinally extending cavity therein, a blade carrier within the cavity and movable longitudinally relative to the handle between an operative position in which a blade carried thereby is exposed for use at an open end of the cavity at a forward end of the handle and an inoperative position in which a blade carried thereby is retracted within the cavity in the handle, and a manually operable slider associated with the blade carrier and passing through a slot in a wall of the handle at an edge thereof, wherein the handle is moulded as a single piece moulding with an integral bridge defining an endless open end to the cavity at said forward end of the handle and wherein the blade carrier is shaped in cross-section to be introduced into the cavity by way of said open end with the slider being formed as a separate part that snap fits to the blade carrier after introduction through the open end of the cavity to form a blade carrier and slider assembly, the scalpel being characterized in that the slider and at least one longitudinally extending edge of the slot are provided with co-operating tooth and notch formations that cooperate to releasably hold the blade carrier and slider assembly in "cilck-stop" manner in the operative and inoperative positions and In that the blade carrier and slider assembly have an innermost, terminal locked position defined by co-operating formations on the slider and edges of the slot, such terminal locked position being one in which the blade carrier is located inwards of the normal inoperative position and from which it is substantially impossible to unlock the blade carrier, at least for practical purposes.

Further features of the invention provide for a plurality of notches to be associated with both the operative and inoperative positions of the blade carrier and slider assembly so that a series of at least two and optionally three or more "click-stops" are associated with each of the operative and inoperative positions such that a person operating the scalpel will know exactly, by feel, and optionally also hearing, the position of the blade carrier in the handle; and for the tooth and notch formations to be adapted such that an audible "click" is created when a tooth engages a notch.

A further feature of the invention provides for the slider to have a pair of transverse tongues with oppositely directed latch formations at their Inner ends for cooperating with cooperant transverse sockets formed in the blade carrier. The tongues are preferably coplanar and spaced apart in the longitudinal direction of the slider.

Preferably, the blade carrier is configured such that it can accept a plurality of different style blades, thereby rendering it more versatile than prior art scalpels.

In order that the invention may be more fully understood one embodiment thereof will now be described with reference to the accompanying drawings.

### BRIEF DESCRIPTION OF THE DRAWINGS

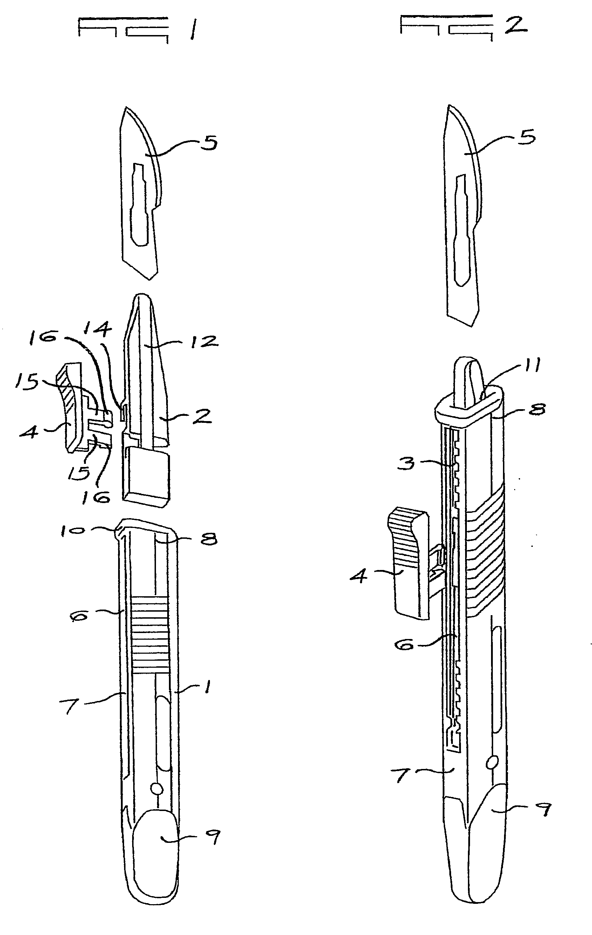

In the drawings:-
- Figure 1: is an exploded perspective view from the side of a scalpel according to the invention but offset such that the top edge of the handle is somewhat visible;
- Figure 2: is a similar view, but more from the top, and showing the scalpel partly assembled;
- Figure 3: is a cross-section taken through the handle of the scalpel;
- Figure 4: is an inverted plan view of the slider showing the tooth and slide block formations formed integral therewith;
- Figure 5: is a detail of the slider in its exploded position relative to the carrier and as illustrated in Figure 2;
- Figure 6: is a detail, partly broken away, and showing the cooperation between tooth and notch formations of the slot and slider with the latter in the operative position;
- Figure 7: is a perspective view of the scalpel from one side of the top thereof showing the slider partly broken away in its fully locked position;
- Figure 8: is a view similar to Figure 6 showing the cooperation between tooth and notch formations of the slot and slider with the latter in the inoperative position; and,
- Figure 9: is a detail similar to Figure 8 but showing the tooth and notch formations in the final locked position of the slider relative to the scalpel handle (in the position illustrated in Figure 7).

### DETAILED DESCRIPTION WITH REFERENCE TO THE DRAWINGS

In the embodiment of the invention illustrated in the drawings, a scalpel comprises a single piece injection moulded plastics handle (1), a blade carrier (2) slidable longitudinally in a longitudinal cavity (3) within the plastics handle, a separately moulded, manually operable slider (4) that combines with the blade carrier to form a blade carrier assembly in the assembled condition, and, for use, a scalpel blade (5) that is fitted to the blade carrier.

The handle has a longitudinally extending slot (6) extending along its operatively top edge (7) from a forward end (8) of the handle towards a rear end (9) thereof and communicating with the cavity inside. An integral bridge (10) at the front end of the top edge forms an endless open end (11) to the cavity and provides dimensional stability to this end for firmly holding the blade carrier in its operative position.

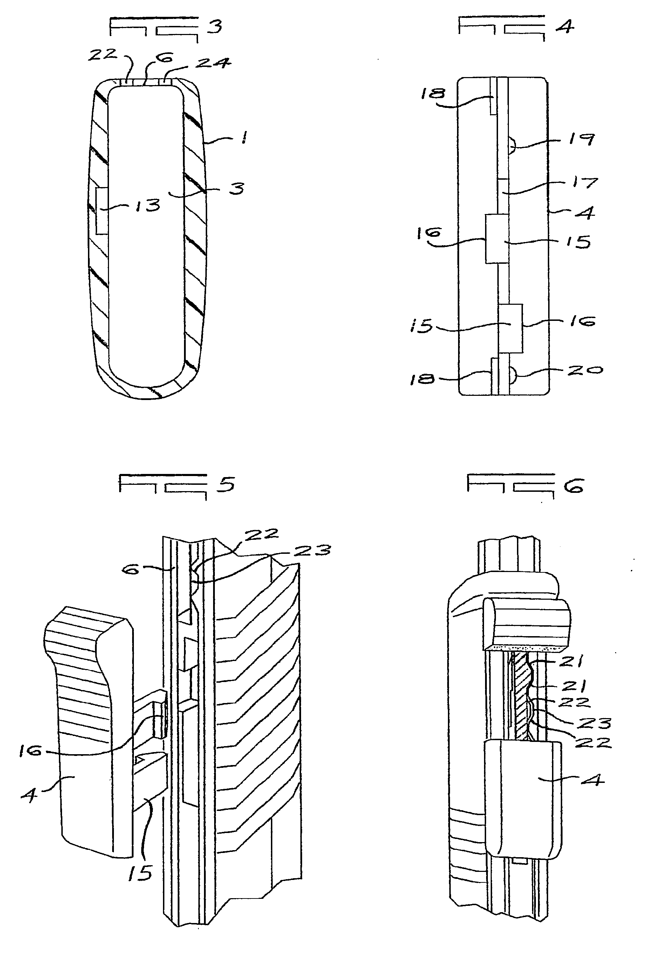

The blade carrier is elongate and has a longitudinally extending ridge (12) that is received in a cooperating groove (13) (see Figure 3) on one side wall of the cavity. The blade carrier is configured to slide longitudinally within the cavity and to receive and support a variety of different scalpel blade types. The blade carrier is clearly shaped, in cross-section, to be introduced through the open end (11) to the cavity.

The blade carrier also has a pair of transverse sockets (14) being configured to receive a pair of transverse tongues (15) extending from the slider, the tongues each having a catch formation (16) at its free end that locks onto the blade carrier in irreversible manner when the tongues are introduced into the sockets with the blade carrier in the cavity. Once this has been achieved, the blade carrier is held captive within the cavity and can be slid forwards and rearwards by manually operating the slider, generally by a person holding the scalpel handle and utilizing the thumb to achieve this.

The slider has, on its upper surface, a longitudinally extending ridge (17) that cooperates with the slot to align the slider correctly relative to it. Extending laterally outwards from the ridge at each end thereof is an integral miniature slide block (18) that cooperates with the one edge of the slot and, on the other side of the ridge, are a forward tooth (19) positioned inwards from the nearer end of the ridge and a rearward tooth (20) located opposite the rear slide block (18).

The teeth (19) and (20) are substantially identical and are of trapezoidal or triangular shape to provide inclined faces (21) to cooperate with the inclined edges (22) to notches (23) formed in the associated edge of the slot at both the front and rear ends thereof. In the case of both the front and rear ends of the slot there are provided a series of four juxtaposed notches for cooperation with the forward tooth (19) and rearward tooth (20) respectively when the slider is at the forward or rear ends of the slot.

The arrangement is such that as the slider is moved towards either of the operative or inoperative positions the respective tooth will engage sequentially with the notches of the series of four and will form a "click-stop" in each position. A person operating the slider will be able to feel these sequential "click-stops" and, with appropriate design, also hear them. It will thus be immediately apparent as to the exact location of the blade relative to the handle.

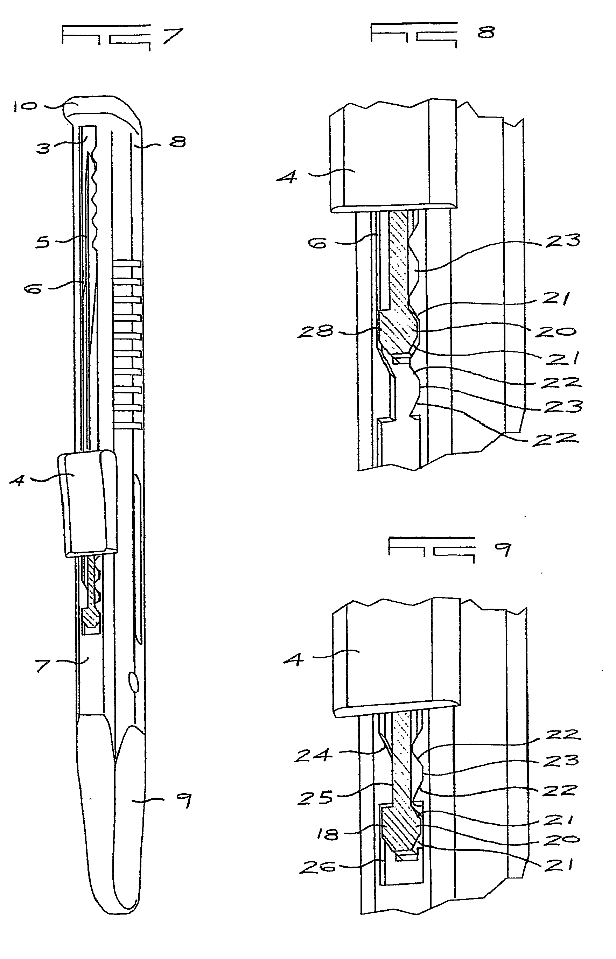

In addition to the above, the rear end of the slot is also provided with a ramp (24) on the side opposite the notches, the ramp communicating with a neck (25) that in turn communicates with a rectangular terminal aperture (26) forming the inner end of the slot. This arrangement is such that when additional force is applied to the slider in a direction towards the rear end of the handle, the slide block (18) and opposite rear tooth (20) are forced together through the neck and into the aperture in an irreversible manner so that the slider is permanently locked in the retracted position. This final position is illustrated clearly in Figure 9.

It will be understood that, in use, the scalpel may be stored and transported with the blade carrier and associated blade in a retracted position with the slider held in one of the "click-stop" retracted positions. This position may also be used during the conduct of operations in between times when the scalpel is required for use. As and when required, the blade carrier can be moved to present the scalpel blade in its operative position with the blade carrier being arrested in a forward "click-stop" position. The position of the blade relative to the handle can be sensed extremely easily by a person using the scalpel. It is also to be noted that the different "click-stop" positions can be used for the purpose of depth control

Once the scalpel has served its purpose and is to be disposed of, the slider is moved to its final locked position so that, to all intents and purposes, it is impossible to use the scalpel again. The scalpel blade is thus held in an extremely safe locked inoperative position for disposal, thereby avoiding the possibility of any so-called sharps injuries.

It will be understood that numerous variations may be made to the embodiment of the invention described above without departing from the scope hereof.

## Claims

1. A scalpel having a handle (1) with a longitudinally extending cavity (3) therein, a blade carrier (2) within the cavity and movable longitudinally relative to the handle between an operative position In which a blade carried thereby is exposed for use at an open end (11) of the cavity at a forward end (8) of the handle and an inoperative position In which a blade carried thereby is retracted within the cavity in the handle, and a manually operable slider (4) associated with the blade carrier and passing through a slot (6) In a wall of the handle at an edge thereof, wherein the handle is moulded as a single piece moulding with an integral bridge (10) defining an endless open end (11) to the cavity at said forward end of the handle and wherein the blade carrier is shaped In cross-section to be introduced into the cavity by way of said open end, the scalpel being **characterized in that** the slider is formed as a separate part that snap fits to the blade carrier after introduction through the open end of the cavity to form a blade carrier and slider assembly, **in that** the slider and at least one longitudinally extending edge of the slot are provided with co-operating tooth (19, 20) and notch (23) formations that cooperate to releasably hold the blade carrier and slider assembly in "click-stop" manner In the operative and inoperative positions and **In that** the blade carrier and slider assembly have an innermost, terminal locked position defined by co-operating formations (25,18, 20) on the slider and edges of the slot, such terminal locked position being one In which the blade carrier is located inwards of the normal inoperative position and from which it is substantially impossible to unlock the blade carrier, at least for practical purposes.

2. A scalpel as claimed in claim 1 in which a plurality of notches are associated with both the operative and inoperative positions of the blade carrier and slider assembly so that a series of at least two, and optionally three or more "click-stops" are associated with each of the operative and inoperative positions.

3. A scalpel as claimed in either one of claims 1 or 2 in which the "click-stops" are configured to create an audible sound upon engagement of a tooth with a notch.

4. A scalpel as claimed In any one of the preceding claims in which the slider has a pair of transverse tongues (15) with oppositely directed catch formations (16) at their inner ends for cooperating with cooperant transverse sockets (14) formed in the blade carrier.

5. A scalpel as claimed In claim 4 in which the tongues are coplanar and spaced apart in the longitudinal direction of the slider.

6. A scalpel as claimed in any one of the preceding claims In which the blade carrier is configured such that it can accept a plurality of different style blades.

## Patentansprüche

1. Skalpell mit einem Griff (1) mit einem dortigen sich in Längsrichtung erstreckenden Hohlraum (3), einem Klingenträger (2) im Hohlraum, der in Längsrichtung bezüglich des Griffs zwischen einer Betriebsposition, in der eine von ihm getragene Klinge zum Gebrauch an einem offenen Ende (11) des Hohlraums an einem vorderen Ende (8) des Griffs freiliegt, und einer Außerbetriebsposition, in der eine von ihm getragene Klinge in den Hohlraum im Griff zurückgezogen ist, beweglich ist, und einem manuell betätigbaren Schieber (4), der dem Klingenträger zugeordnet ist und durch einen Schlitz (6) in einer Wand des Griffs an einem Rand davon geht, wobei der Griff als ein einteiliges Formteil mit einer integralen Brücke (10) geformt ist, die ein endloses offenes Ende (11) zum Hohlraum am vorderen Ende des Griffs definiert, und wobei der Klingenträger im Querschnitt so ausgebildet ist, dass er über das offene Ende in den Hohlraum eingeführt wird, **dadurch gekennzeichnet, dass** der Schieber als ein getrenntes Teil ausgebildet ist, das nach der Einführung durch das offene Ende des Hohlraums in den Klingenträger einrastet, so dass eine Anordnung aus Klingenträger und Schieber gebildet wird, dass der Schieber und mindestens ein sich in Längsrichtung erstreckender Rand des Schlitzes mit zusammenwirkenden Zahn-(19, 20) und Kerbengebilden (25) versehen sind, die zusammenwirken, um die Anordnung aus Klingenträger und Schieber lösbar rastungsartig in der Betriebs- und der Außerbetriebsposition zu halten, und dass die Anordnung aus Klingenträger und Schieber eine zu innerst liegende verriegelte Endposition hat, die durch zusammenwirkende Gebilde (25, 18, 20) am Schieber und Ränder des Schlitzes definiert ist, wobei eine derartige verriegelte Endposition eine solche ist, in der der Klingenträger von der normalen Außerbetriebsposition nach innen angeordnet ist und von der aus es, zumindest praktisch genommen, im Wesentlichen unmöglich ist, den Klingenträger zu entriegeln.

2. Skalpell nach Anspruch 1, wobei mehrere Kerben sowohl der Betriebs- als auch der Außerbetriebsposition der Anordnung aus Klingenträger und Schieber zugeordnet sind, so dass der Betriebs- und der Außerbetriebsposition jeweils eine Reihe von mindestens zwei und wahlweise drei oder mehr Rastungen zugeordnet sind.

3. Skalpell nach Anspruch 1 oder 2, wobei die Rastungen so konfiguriert sind, dass sie beim Eingriff eines Zahns in einer Kerbe einen hörbaren Ton erzeugen.

4. Skalpell nach einem der vorhergehenden Ansprüche, wobei der Schieber ein Paar Querzungen (15) mit entgegengesetzt gerichteten Arretierungsgebilden (16) an ihren inneren Enden hat, um mit im Klingenträger ausgebildeten zusammenwirkenden Querbuchsen (14) zusammenzuwirken.

5. Skalpell nach Anspruch 4, wobei die Zungen koplanar und in der Längsrichtung des Schiebers beabstandet sind.

6. Skalpell nach einem der vorhergehenden Ansprüche, wobei der Klingenträger so konfiguriert ist, dass er mehrere Klingen unterschiedlicher Art aufnehmen kann.

## Revendications

1. Scalpel doté d'une poignée (1) comprenant, à l'intérieur une cavité (3) s'étendant dans le sens de la longueur, un porte-lame (2) positionné à l'intérieur de la cavité et mobile dans le sens de la longueur par rapport à la poignée entre une position de fonctionnement dans laquelle une lame supportée est exposée pour être utilisée au niveau d'une extrémité ouverte (11) de la cavité, au niveau d'une extrémité avant (8) de la poignée, et une position de non-fonctionnement dans laquelle une lame supportée est rétractée à l'intérieur de la cavité dans la poignée ainsi qu'un curseur (4) actionnable manuellement associé au porte-lame et traversant une fente (6) dans une paroi de la poignée au niveau d'une arête de celle-ci, dans lequel la poignée est moulée à la façon d'un moulage d'un seul tenant avec un pont intégral (10) définissant une extrémité ouverte (11) sans fin ouverte en direction de la cavité au niveau de ladite extrémité avant de la poignée et dans lequel le porte-lame est formé en section transversale de façon à être introduit dans la cavité au niveau de ladite extrémité ouverte, le scalpel étant **caractérisé en ce que** le curseur prend la forme d'une partie séparée qui s'emboîte par pression avec le porte-lame après avoir été introduit à travers l'extrémité ouverte de la cavité pour former un ensemble porte-lame et curseur, **en ce que** le curseur et au moins une arête s'étendant dans le sens de la longueur de la fente sont pourvus d'une formation de dents (19, 20) et entaille (23) en coopération qui coopèrent pour maintenir de façon libérable l'ensemble porte-lame et curseur par « encliquetage d'arrêt » dans les positions de fonctionnement et de non-fonctionnement et **en ce que** l'ensemble porte-lame et curseur a une position verrouillée terminale la plus reculée à l'intérieur définie par des formations coopérantes (25, 18, 20) sur le curseur et les arêtes de la fente, une telle position verrouillée terminale étant une position dans laquelle le porte-lame est positionné vers l'intérieur de la position normale de non-fonctionnement et depuis laquelle il est sensiblement impossible de déverrouiller le porte-lame, au moins à des fins pratiques.

2. Scalpel selon la revendication 1, dans lequel une pluralité d'entailles sont associées tant à la position de fonctionnement que de non-fonctionnement de l'ensemble porte-lame et curseur de sorte qu'une série d'au moins deux et, optionnellement, de trois « encliquetages d'arrêt » ou plus sont associés à chacune des positions de fonctionnement et de non-fonctionnement.

3. Scalpel selon l'une quelconque des revendications 1 ou 2, dans lequel les « encliquetages d'arrêt » sont configurés pour créer un son audible lors de l'emboîtement d'une dent avec une entaille.

4. Scalpel selon l'une quelconque des revendications précédentes, dans lequel le curseur comprend une paire de languettes (15) transversales dotées de formations de fermeture (16) opposées au niveau de leurs extrémités intérieures prévues pour coopérer avec les raccords (14) transversaux coopérants formés dans le porte-lame.

5. Scalpel selon la revendication 4, dans lequel les languettes sont coplanaires et espacées les unes par rapport aux autres dans la direction longitudinale du curseur.

6. Scalpel selon l'une quelconque des revendications précédentes, dans lequel le porte-lame est configuré de telle sorte qu'il peut recevoir une pluralité de lames de différents types.
